(19) Europäisches Patentamt
European Patent Office
Office européen des brevets

(11) Publication number : 0 408 506 B1

# (12) EUROPEAN PATENT SPECIFICATION

(45) Date of publication of patent specification : 17.08.94 Bulletin 94/33

(51) Int. Cl.[5] : **C07D 249/08**, C07D 249/18, C10M 133/44

(21) Application number : 90810512.5

(22) Date of filing : 05.07.90

## (54) New triazole compounds.

(30) Priority : 14.07.89 GB 8916195

(43) Date of publication of application : 16.01.91 Bulletin 91/03

(45) Publication of the grant of the patent : 17.08.94 Bulletin 94/33

(84) Designated Contracting States : BE DE ES FR GB IT NL

(56) References cited :
EP-A- 0 365 476
GB-A- 1 563 199
US-A- 4 153 565

(56) References cited :
JOURNAL OF ORGANIC CHEMISTRY, vol. 54, no. 26, 22nd December 1989, pages 6022-6029, Washington, DC, US; A.R. KATRITZKY et al.: "Reactions of 1-(alpha-alkoxyalkyl)-and 1-(alpha-(aryloxy)alkyl)benzotriazoles with the Grignard reagents. A new and versatile method for the preparation of ethers"

(73) Proprietor : CIBA-GEIGY AG
Klybeckstrasse 141
CH-4002 Basel (CH)

(72) Inventor : O'Neil, Robert Montgomery, Dr.
54, Derwent Road
Flixton, Manchester M31 2UA (GB)

EP 0 408 506 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to new triazole compounds, to their production and to their use as metal deactivators and/or antioxidants in organic material such as mineral oils.

In GB-A-2002772, there are described compounds, useful as metal deactivators in lubricants, and having the formula:

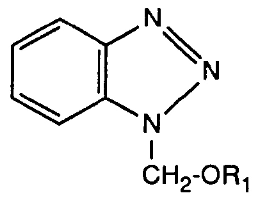

wherein $R_1$ is 1-18C alkyl, 3-18C alkenyl, 5-12C cycloalkyl or 6-10C aryl, each optionally substituted with one or more 1-12C alkyl or 7-9C aralkyl groups.

US-A-4153565 discloses lubricant compositions containing, as antioxidants and corrosion inhibitors, the adduct of i) benzotriazole, optionally ring-substituted with a 1-12C hydrocarbyl group, and ii) an alkyl vinyl ether or a vinyl ester of a hydrocarbylcarboxylic acid of formula

$$R_{II}\text{-}CH{=}CH\text{-}O\text{-}R_{III}$$

or

$$R_{II}\text{-}CH{=}CH\text{-}O\text{-}C({=}O){=}R_{IV}$$

in which $R_{II}$ is hydrogen or a 1-8C alkyl group, $R_{III}$ is 1-18C alkyl and $R_{IV}$ is alkyl, aryl, alkaryl or aralkyl containing 1-18C atoms.

Also known, in US-A-4260501, are lubricant compositions comprising the adducts of US-A-4153565 together with an alkyl dimercapto thiadiazole, especially tertiary octyl 2,5-dimercapto thiadiazole, more especially tertiary octyl 2,5-dimercapto-1,3,4-thiadiazole.

We have now found that certain N-substituted triazoles provide improved metal deactivator and antioxidant properties when incorporated into organic material e.g. mineral oils.

Accordingly, the present invention provides new triazole compounds having the formula I:

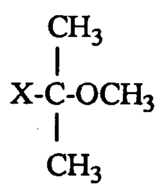

(I)

wherein X is the triazole residue of formula II:

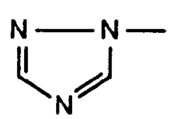

(II)

or the benzotriazole residue of formula III or IIIA:

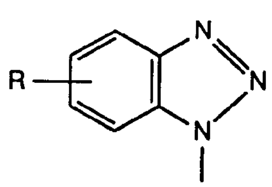 (III) 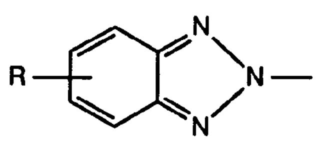 (IIIA)

in which R is hydrogen or methyl.

The compounds of formula I may be produced by reacting, in the presence of an acid catalyst, 2-methoxypropene of formula IV:

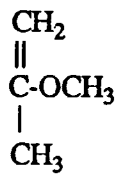

(IV)

with a triazole compound having the formula V, VI or VIA:

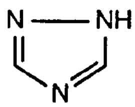 (V) 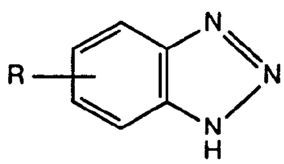 (VI) ⇌ 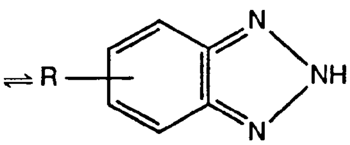 (VIA)

in which R has its previous significance.

Due to the tautomerism between formulae VI and VIA, two possible products may be obtained, represented by formulae III and IIIA, respectively.

The process is conveniently performed in an inert solvent, at ambient or elevated temperature. Solvents which may be used include aromatic solvents such as benzene, toluene or xylene; cyclohexane; carbon tetrachloride; or dioxan.

The amount of 2-methoxypropene reactant of formula IV used is preferably that which is stoichiometrically required for complete reaction with the triazole compound of formula V or VI, or a slight excess over the stoichiometric amount required. Preferably 1-10%, especially 5-10% molar excess of 2-methoxypropene is used relative to the triazole component.

Acid catalysts for use in the process include e.g. sulphuric acid, phosphoric acid, an acid ion-exchange resin, e.g. Amberlyst® 15, bentonite, montmorillonite, Fuller's earth or p-toluene sulphonic acid.

Most preferably, the reaction may be effected by the dropwise addition of 2-methoxypropene to a solution of the triazole compound of formula V or VI, dissolved in toluene or dioxan, at 25 to 60°C, in the presence of a catalytic amount of p-toluene sulphonic acid, preferably 1-10 weight%, especially 1-5 weight%, based on the triazole component.

The product of formula I may be isolated from the reaction mixture by removal of catalyst and reaction solvent, followed by vacuum distillation of the residue.

According to the present invention, there is also provided a composition comprising an organic material and, as metal deactivator and/or antioxidant, at least one compound having the formula I, as hereinbefore defined.

The organic material component of the compositions of the present invention may be any organic material which is susceptible to degradation in the presence of degradants such as metals and/or oxygen. Examples of such organic materials are mineral oils, synthetic oils, plastics and other polymers.

Of particular interest are lubricants which are of mineral oil origin or are synthetic oils e.g. carboxylic acid esters, especially those intended for use at temperatures at or above 200°C.

Examples of carboxylic acid ester synthetic lubricants include those based on a diester of a dibasic acid and a monohydric alcohol e.g. dioctyl sebacate or dinonyl adipate; or a triester of trimethylol propane and a monobasic acid or mixture of such acids e.g. trimethylol propane tripelar- gonate, trimethylol propane tricaprylate or mixtures of these; on a tetraester of pentaerythritol and a monobasic acid or a mixture of such acids e.g. pentaerythritol tetracaprylate; or on complex esters derived from monobasic acids, dibasic acids and polyhydric alcohols e.g. a complex ester derived from trimethylolpropane, caprylic acid and sebacic acid; or mixtures of one or more of such carboxylic acid esters.

Other synthetic lubricant bases are those described e.g. in "Schmiermittel-Taschenbuch" (Huethig Verlag, Heidelberg 1974), e.g. phosphates, glycols, polyglycols, polyalkylene glycols and poly-alpha olefins.

Mineral oil-based lubricant bases are preferred.

The compositions of the present invention preferably contain 0.001 to 5.0%, more preferably 0.02 to 1.0% by weight of a compound of formula I, based on the weight of the organic material.

In addition to the compound of formula I, the organic material compositions according to the present invention may contain, in order to improve the operating properties of the organic material, further additives. Such further additives include, in the case of preferred lubricant organic materials, e.g. further antioxidants

e.g. phenolic antioxidants, amine antioxidants, or other antioxidants, further metal deactivators, rust inhibitors, viscosity-index improvers, pour-point depressants, dispersants/-surfactants, and anti--wear additives.

The compounds of formula I, when used alone, exert an excellent metal deactivating effect on working metal surfaces e.g. engine parts, especially of iron or, in particular copper, in contact with an organic material containing a metal degradant such as sulphur.

When, however, the organic material per se is the primary target for degradation e.g. when used in the presence of adventitious traces of metals such as iron or copper, and/or oxygen and/or hydroperoxides, then it is very much preferred to use the compound of formula I in combination with a further antioxidant.

Examples of phenolic antioxidants

1. Alkylated Monophenols

2,6-Di-tert-butyl-4-methylphenol, 2,6-di-tert-butylphenol, 2-tert-butyl-4,6-dimethylphenol, 2,6-di-tert-butyl-4-ethylphenol, 2,6-di-tert-butyl-4-n-butylphenol, 2,6-di-tertbutyl-4-i-butylphenol, 2,6-di-cyclopentyl-4-methylphenol, 2-(β-methylcyclohexyl)4,6-di-methylphenol, 2,6-di-octadecyl-4-methylphenol, 2,4,6-tri-cyclohexylphenol, 2,6-di-tert-butyl-4-methoxymethylphenol, o-tert-butylphenol.

2. Alkylated Hydroquinones

2,6-Di-tert-butyl-4-methoxyphenol, 2,5-di-tert-butyl-hydroquinone, 2,5-di-tert-amylhydroquinone, 2,6-diphenyl-4-octadecyloxyphenol.

3. Hydroxylated Thiodiphenylethers

2,2′-Thio-bis-(6-tert-butyl-4-methylphenol), 2,2′-thio-bis-(4-octylphenol), 4,4′-thio-bis-(6-tert-butyl-3-methylphenol), 4,4′-thio-bis-(6-tert-butyl-2-methylphenol).

4. Alkylidene-Bisphenols

2,2′-Methylene-bis-(6-tert-butyl-4-methylphenol), 2,2′-methylene-bis-(6-tert-buryl-4-ethylphenol), 2,2′-methylene-bis-(4-methyl-6-(α-methylcyclohexyl)-phenol), 2,2′-methylene-bis-(4-methyl-6-cyclo-hexylphenol), 2,2′-methylene-bis-(6-nonyl-4-methylphenol), 2,2′-methylene-bis-(4,6-di-tert-butyl- phenol), 2,2′-ethylidene-bis-(4,6-di-tert-butylphenol), 2,2′-ethylidene-bis-(6-tert-butyl-4- or -5-isobutylphenol), 2,2′-methylene-bis-(6-α-methylbenzyl-4-nonylphenol), 2,2′-methylene-bis-(6-(α,α-dimethylbenzyl)-4-nonylphenol), 4,4′-methylene-bis-(2,6-di-tert-butylphenol), 4,4′-methylene-bis-(6-tert-butyl-2-methylphenol), 1,1′-bis-(5-tert-butyl-4-hydroxy-2-methylphenol)-butane, 2,6-di-(3-tert-butyl-5-methyl-2-hydroxybenzyl)-4-methylphenol, 1,1,3-tris-(5-tert-butyl-4-hydroxy-2-methylphenyl)-3-n-dodecyl)-mercapto-butane, ethyleneglycol-bis-[3,3-bis-(3′-tert-butyl-4′-hydroxyphenyl)-butyrate], bis-(3-tert-butyl-4-hydroxy-5-methylphenyl)-dicyclo-pentadiene, bis-[2-(3′-tert-butyl-2′-hydroxy-5′-methyl-benzyl)-6-tert-butyl-4-methyl-phenyl]-terephthalate.

5. Benzyl Compounds

1,3,5-Tri-(3,5-di-tert-butyl-4-hydroxybenzyl)2,4,6-tri-methyl-benzene, bis-(3,5-di-tert-butyl-4-hydroxybenzyl)-sulfide, 3,5-di-tert-butyl-4-hydroxybenzyl-mercaptoacetic acid-isooctylester, bis-(4-tert-butyl-3-hydroxy-2,6-dimethylbenzyl)-dithiolterephthalate, 1,3,5-tris-(3,5-di-tert-butyl-4-hydroxy-benzyl)-isocyanurate, 1,3,5-tris-(4-tert-butyl-3-hydroxy-2,6-dimethylbenzyl)-isocyanurate, 3,5-di-tert-butyl-4-hydroxybenzyl-phosphonic acid-dioctadecylester, 3,5-di-tert-butyl-4-hydroxy-benzyl-phosphonic acid-monoethylester, calcium-salt.

6. Acylaminophenols

4-Hydroxy-lauric acid anilide, 4-hydroxy-stearic acid anilide, 2,4-bis-octylmercapto-6-(3-,5-di-tert-butyl-4-hydroxy-anilino)-s-triazine, N-(3,5-di-tert-butyl-4-hydroxyphenyl)-carbamic acid octyl ester.

7. Esters of β-(3,5-Di-tert-butyl-4-hydroxyphenol)-propionic acid

with mono- or polyhydric alcohols, for example with methanol, diethyleneglycol, octadecanol, triethyleneglycol, 1,6-hexanediol, pentaerythritol, neopentylglycol, tris-hydroxyethyl-isocyanurate, thiodiethyleneglycol, bis-hydroxyethyl-oxalic acid diamide.

8. Esters of β-(5-tert-butyl-4-hydroxy-3-methylphenyl)-propionic acid

with mono- or polyhydric alcohols, for example with methanol, diethyleneglycol, octadecanol, triethyleneglycol, 1 ,6-hexanediol, pentaerythritol, neopentylglycol, tris-hydroxyethyl-isocyanurate, thiodiethyleneglycol, di-hydroxyethyl-oxalic acid diamide.

9. Amides of β-(3,5-Di-tert-butyl-4-hydroxyphenyl)-propionic acid for example

N,N'-Bis(3,5-di-tert-butyl-4-hydroxyphenylpropionyl)- hexamethyl- enediamine, N,N'-bis-(3,5-di-tert-butyl-4--hydroxyphenylpropionyl)-trimethylene-diamine, N,N'-bis-- (3,5-di-tert-butyl-4- hydroxyphenylpropionyl)-hydrazine.

Examples of amine antioxidants:

N,N'-Di-isopropyl-p-phenylenediamine, N,N'-di-sec.-butyl-p-phenylenediamine, N,N'-bis(1 ,4-dimethyl-pentyl)-p-phenylenediamine,N,N'-bis( 1-ethyl-3-methylpentyl)-p-phenylenediamine, N,N'-bis(1-methyl-heptyl)-p-phenylenediamine, N,N'-dicyclohexyl-p-phenylenediamine, N,N'-diphenyl-p-phenylenediamine, N,N'-di-(naphthyl-2-)-p-phenylenediamine, N-isopropyl-N'-phenyl-p-phenylenediamine, N-(1,3-dimethyl-butyl)-N'-phenyl-p-phenylene-diamine, N-(1-methyl-heptyl)-N'-phenyl-p-phenylenediamine, N-cyclohexyl-N'-phenyl-p-phenylenediamine, 4-(p-toluene-sulfon-amido)-diphenylamine, N,N'-dimethyl-N,N'-di-sec-butyl-p-phenylenediamine, diphenylamine, N-allyldiphenylamine, 4-isopropoxy-diphenylamine, N-phenyl-1-naphthylamine, N-phenyl-2-naphthyl-amine, octylated diphenylamine, e.g. p,p'-di-tert-octyldiphenylamine, 4-n-butylaminophenol, 4-butyrylaminophenol, 4-nonanoylaminophenol, 4-dodecanoylaminophenol, 4-octadecanoylaminophenol, di-(4-methoxy-phenyl)-amine, 2,6-di-tert-butyl-4- dimethylamino-methyl-phenol, 2,4'-diamino-diphenylmethane, 4,4'-diamino-diphenylmethane, N,N,N',N'-tetramethyl-4,4'-diamino-diphenylmethane, 1 ,2-di-(phenylamino)-ethane, 1,2-di-[2-methyl-phenyl)amino]-ethane, 1 ,3-di-(phenyl- amino)-propane, (o-tolyl)-biguanide, di-[4-(1',3'-dimethylbutyl)-phenyl]amine, tert-octylated N-phenyl-1-naphthyl-amine, mixture of mono- and dialkylated tert-butyl-/tert-octyldiphenylamines, 2,3-dihydro-3,3-dimethyl-4H-1,4-benzothiazine, phenothiazine, n-allyl-phenothiazine.

Examples for other antioxidants:

Aliphatic or aromatic phosphites, esters of thiodipropionic acid or of thiodiacetic acid, or salts of dithiocarbamic or dithiophosphoric acid.

Examples of metal deactivators, for example for copper, are:

Triazoles, benzotriazoles and derivatives thereof, tolutriazole and derivatives thereof, 2-mercaptobenzothiazole, 2,5-dimercaptothiadiazole, 5,5'-methylene-bis-benzotriazole, 4,5,6,7-tetrahydro-benzotriazole, salicylidene-propylenediamine and salicylaminoguanidine and salts thereof.

Examples of rust inhibitors are:

a) Organic acids, their esters, metal salts and anhydrides, e.g. N-oleoyl-sarcosine, sorbitan-mono-oleate, lead-naphthenate, alkenyl-succinic acids and -anhydrides, e.g. dodecenyl-succinic acid anhydride, succinic acid partial esters and amides, 4-nonylphenoxy-acetic acid.
b) Nitrogen-containing compounds, e.g.
I Primary, secondary or tertiary aliphatic or cycloaliphatic amines and amine-salts of organic and inorganic acids, e.g. oil-soluble alkylammonium carboxylates
II Heterocyclic compounds, e.g.
substituted imidazolines and oxazolines.
c) Phosphorus-containing compounds, e.g.
Amine salts of phosphonic acid or phosphoric acid partial esters, zinc dialkyldithio phosphates.
d) Sulphur-containing compounds, e.g.
Barium-dinonylnaphthalene-n-sulphonates, calcium petroleum sulphonates.

Examples of viscosity-index improvers are:

Polyacrylates, polymethacrylates, vinylpyrrolidone/methacrylate-co-polymers, polyvinylpyrrolidones, polybutenes, olefin-copolymers, styrene/acrylate-copolymers, polyethers.

Examples of pour-point depressants are:

Polymethacrylates, alkylated naphthalene derivatives.

Examples of dispersants/surfactants are:

Polybutenylsuccinic acid-amides or -imides, polybutenylphosphonic acid derivatives, basic magnesium-, calcium-, and barium-sulfonates and -phenolates.

Examples of anti-wear additives are:

Sulphur- and/or phosphorus- and/or halogen-containing compounds e.g. sulphurised vegetable oils, zinc dialkyldithio-phosphates, tritolyl-phosphate, chlorinated paraffins, alkyl- and aryldi- and tri-sulphides, triphenylphosphorothionate.

When the organic material is an organic material liable to degradation by oxidation, e.g. a lubricant composition, one particular preferred class of co-additives for use in conjunction with the compounds of formula I, comprises phenolic or amine-type antioxidants, especially amine-type antioxidants e.g. diphenylamine, octylated diphenylamine, N-phenyl-1-naphthylamine and N-(octylated-phenyl)-1-naphthyl-amine, with which the compounds of formula I exhibit a synergistic effect.

The following Examples further illustrate the present invention.

Example 1: 2-[1H-Benzotriazolyl]-2′-methoxypropane

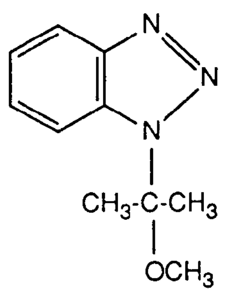

A solution of benzotriazole (23.8 g; 0.20 mole) in toluene (200 mi) containing p-toluene sulphonic acid (0.24 g) is maintained at 60°C and treated, dropwise, over 30 minutes with 2-methoxypropene (15.8 g; 0.22 mole). After complete addition, the mixture is maintained at 60°C for a further 30 minutes. The mixture is allowed to cool to ambient temperature, washed with dilute sodium carbonate solution, then with water and, finally, dried over anhydrous magnesium sulphate. The dried extract is filtered and evaporated to dryness on a rotary evaporator to yield the crude product as a pale yellow oil (31.7 g; 83 %). The pure product is obtained as a colourless oil by vacuum distillation, bp 113°/30 Pa.

Analysis: Found C 62.29 %; H 6.74 %; N 22.33 %

$C_{10}H_{13}N_3O$ Requires C 62.81 %; H 6.85 %; N 21.97 %

Example 2: 2-[1H-Tolyltriazolyl]-2'-methoxypropane

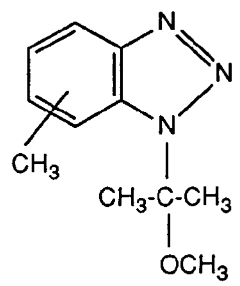

A solution of tolyltriazole (26.6 g; 0.2 mole) in toluene (200 mi) containing p-toluene sulphonic acid (0.27 g) is maintained at 25-35°C whilst being treated, dropwise, over 30 minutes with 2-methoxypropene (15.8 g; 0.22 mole). After complete addition, the mixture is heated to 60°C and maintained at this temperature for 5 minutes. The mixture is allowed to cool to room temperature, washed with dilute sodium carbonate solution, then with water and, finally, dried over anhydrous magnesium sulphate. The dried extract is filtered and evaporated to dryness to yield the crude product as a pale yellow oil (37.1 g; 93 %). The pure product is obtained as a pale yellow oil by vacuum distillation, bp 120°/30 Pa.
Analysis: Found C 64.02 %; H 7.69 %; N 20.66 %
$C_{11}H_{15}N_3O$ Requires C 64.36 %; H 7.37 %; N 20.48 %

Example 3: 2-[1H-1,2,4-Triazolyl]-2'-methoxypropane

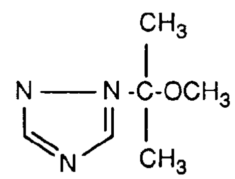

A solution of 1,2,4-triazole (6.9 g; 0.1 mole) in dioxan (200 mi) containing p-toluene sulphonic acid (0.20 g) is maintained at 50°C and treated, dropwise, over 15 minutes with 2-methoxypropene (7.9 g; 0.11 mole). After complete addition, the mixture is heated to 100°C and maintained at this temperature for 5 minutes. The mixture is allowed to cool to ambient temperature and is then evaporated on a rotary evaporator. The oily product thus obtained is then extracted into ether and the extract washed with dilute sodium carbonate solution. The extract is dried over anhydrous magnesium sulphate and then Filtered and evaporated to dryness on a rotary evaporator to yield the crude product as a pale yellow oil (11.7 g; 79 %). The pure product is obtained as a colourless oil by vacuum distillation, bp 60°/100 Pa.
Analysis: Found C 50.06 %; H 7.86 %; N 29.78 %
$C_6H_{11}N_3O$ Requires C 51.05 %; H 7.85 %; N 29.78 %

Examples 4 to 6

(Modified) ASTM D-130 Copper Strip Test

A 0.05% solution of the test compound is prepared in a turbine quality mineral oil of viscosity 26.2 $mm^2$/-at 40°C, 4.8 $mm^2$/s at 100°C and S-content of 0.54% in which 50 ppm of elemental sulphur has been dissolved.

A copper strip (60 x 10 x 1 mm) is polished with 100 grade silicon carbide grit which has been picked up on cotton wool wetted with petroleum ether. The polished strip is then immediately totally immersed in the prepared solution, which is maintained at 100°C for 2 hours. After this time, the strip is removed, washed with petroleum ether, dried and its colour is compared with those of the ASTM D130 Copper Strip Corrosion Standard Chart.

The results are summarised in the following Table:

Modified ASTM D-130 Copper Strip Test

| Example | Test compound | ASTM D-130 Rating |
|---|---|---|
| - | none (control) | 3B |
| 4 | Product Ex. 1 | 1A |
| 5 | Product Ex. 2 | 1A |
| 6 | Product Ex. 3 | 3B |

A rating of 1 denotes a slight tarnish; a rating of 2 a moderate tarnish; a rating of 3 a dark tarnish; and a rating of 4 severe corrosion. otters A, B, C and D are used to indicate shadings within the broad numerical values. The results in the Table demonstrate the excellent test results achieved using compositions according to the present invention.

Examples 7 to 12

Rotary Bomb Oxidation Test ASTM D-2272

A 0.05% solution of the test compound is prepared in a turbine quality mineral oil of viscosity 26.2 $mm^2/s$ at 40°C, 4.8 $mm^2/s$ at 100°C and 5-content of 0.54% which may also contain either a phenolic or aminic antioxidant, or both.

The time taken for the oxygen pressure in the bomb to drop more than 175 kPa below the maximum pressure is recorded.

The results obtained are set out in the following Table:

| Example | Test Compound Product of Example No. | Antioxidant A | Antioxidant B | RBOT mins to 175 kPa pressure drop |
|---|---|---|---|---|
| control | Base oil alone | | | 25 mins |
| control | none | 0.10 % | - | 65 mins |
| control | none | - | 0.10 % | 85 mins |
| 7 | 1 | 0.10 % | - | 540 mins |
| 8 | 1 | - | 0.10 % | 310 mins |
| 9 | 2 | 0.10 % | - | 415 mins |
| 10 | 2 | - | 0.10 % | 555 mins |
| 11 | 3 | 0.10 % | - | 420 mins |
| 12 | 3 | - | 0.10 % | 1295 mins |

Antioxidant A is a commercially available mxiture of tert-butylated phenols.

Antioxidant B is a commercially available di-tert-octylated diphenylamine.

The results in the Table indicate that, when used in combination with a further amine or phenolic antioxidant, the compounds of formula I impart synergistic antioxidant properties to the lubricant compositions of the invention.

**Claims**

**Claims for the following Contracting States : BE, DE, FR, GB, IT, NL**

**1.** A triazole compound having the formula I

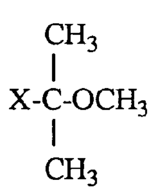

(I)

wherein X is the triazole residue of formula II

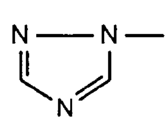

(II)

or the benzotriazole residue of formula III or IIIA

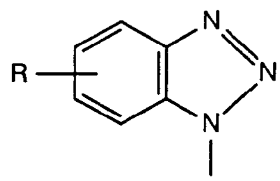 (III) 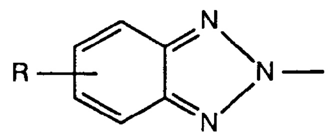 (IIIA)

in which R is hydrogen or methyl.

2. A process for the production of a triazole compound of formula I, as defined in claim 1, comprising reacting, in the presence of an acid catalyst, 2-methoxypropene of formula IV:

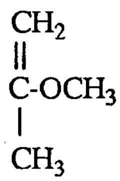 (IV)

with a triazole compound having the formula V or VI:

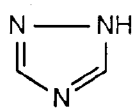 (V) 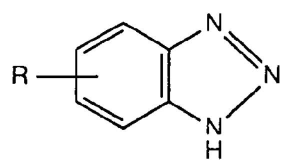 (VI)

in which R is as defined in claim 1.

3. A process according to claim 2 wherein the acid catalyst is sulphuric acid, phosphoric acid, an acid ion-exchange resin, bentonite, montmorillonite, Fullers' earth or p-toluene sulphonic acid.

4. A composition comprising an organic material and as metal deactivator and/or antioxidant, at least one compound having the formula I, as defined in claim 1.

5. A composition according to claim 4 wherein the organic material is a lubricant based on mineral oil or on synthetic oils.

6. A composition according to claim 5 wherein the lubricant is based on mineral oil.

7. A composition according to claim 4 wherein 0.001 to 5.0 % by weight of a compound of formula I is present, based on the weight of the organic material.

8. A composition according to claim 7 wherein 0.02 to 1.0 % by weight of a compound of formula I is present, based on the weight of the organic material.

9. A composition according to claim 4 comprising one or more further additives known to improve the operating properties of the organic material.

10. A composition according to claim 9 wherein the organic material is a lubricant and the further additive is one or more of a further antioxidant, a further metal deactivator, a rust inhibitor, a viscosity-index improver, a pour-point depressant, a dispersant/surfactant and an anti-wear additive.

11. A composition according to claim 10 wherein the further additive comprises a phenolic or amine-type antioxidant.

12. A composition according to claim 11 wherein the further additive is diphenylamine, octylated diphenylamine, N-phenyl-1-naphthylamine or N-(octylated-phenyl)-1-naphthylamine.

**Claims for the following Contracting State: ES**

1. A process for the production of a triazole compound of formula I,

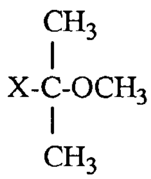

(I)

wherein X is the triazole residue of formula II

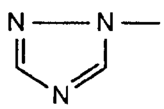

(II)

or the benzotriazole residue of formula III or IIIA

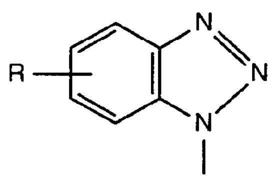

(III)

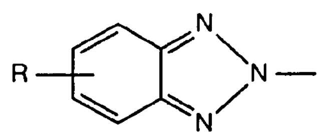

(IIIA)

in which R is hydrogen or methyl, comprising reacting, in the presence of an acid catalyst, 2-methoxypropene of formula IV:

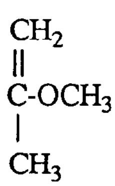

(IV)

with a triazole compound having the formula V or VI:

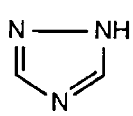

(V)

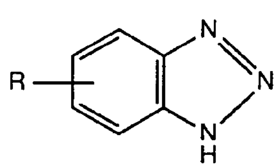

(VI)

in which R is as defined above.

2. A process according to claim 1 wherein the acid catalyst is sulphuric acid, phosphoric acid, an acid ion-exchange resin, bentonite, montmorillonite, Fullers' earth or p-toluene sulphonic acid.

3. A composition comprising an organic material and as metal deactivator and/or antioxidant, at least one compound having the formula I, as defined in claim 1.

**4.** A composition according to claim 3 wherein the organic material is a lubricant based on mineral oil or on synthetic oils.

**5.** A composition according to claim 4 wherein the lubricant is based on mineral oil.

**6.** A composition according to claim 3 wherein 0.001 to 5.0 % by weight of a compound of formula I is present, based on the weight of the organic material.

**7.** A composition according to claim 6 wherein 0.02 to 1.0 % by weight of a compound of formula I is present, based on the weight of the organic material.

**8.** A composition according to claim 3 comprising one or more further additives known to improve the operating properties of the organic material.

**9.** A composition according to claim 8 wherein the organic material is a lubricant and the further additive is one or more of a further antioxidant, a further metal deactivator, a rust inhibitor, a viscosity-index improver, a pour-point depressant, a dispersant/surfactant and an anti-wear additive.

**10.** A composition according to claim 9 wherein the further additive comprises a phenolic or amine-type antioxidant.

**11.** A composition according to claim 10 wherein the further additive is diphenylamine, octylated diphenylamine, N-phenyl-1-naphthylamine or N-(octylated-phenyl)-1-naphthylamine.

**Patentansprüche**

**Patentansprüche für forlgende Vertragsstaaten : BE, DE, FR, GB, IT, NL**

**1.** Triazolverbindung der Formel I

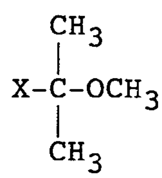

(I)

worin X den Triazolrest der Formel II

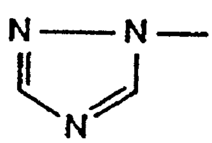

(II)

oder den Benzotriazolrest der Formel III oder IIIA bedeutet

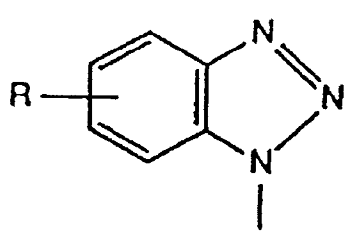

(III)

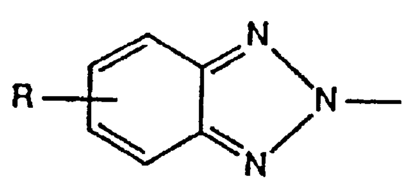

(IIIA)

worin R Wasserstoff oder Methyl bedeutet.

2. Verfahren zur Herstellung einer Triazolverbindung der Formel I nach Anspruch 1, umfassend die Umsetzung von 2-Methoxypropen der Formel IV:

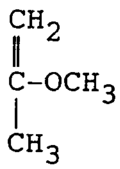

(IV)

mit einer Triazolverbindung der Formel V oder VI:

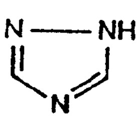

(V)

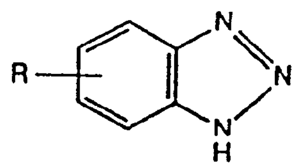

(VI)

worin R wie in Anspruch 1 definiert ist, in Gegenwart eines sauren Katalysators.

3. Verfahren nach Anspruch 2, wobei der saure Katalysator Schwefelsäure, Phosphorsäure, ein saures Ionenaustauscherharz, Bentonit, Montmorillonit, Fuller's Erde oder p-Toluolsulfonsäure ist.

4. Zusammensetzung, umfassend ein organisches Material und als Metalldesaktivator und/oder Antioxidans mindestens eine Verbindung der Formel I nach Anspruch 1.

5. Zusammensetzung nach Anspruch 4, wobei das organische Material ein Schmiermittel auf der Grundlage von Mineralöl oder synthetischen Ölen ist.

6. Zusammensetzung nach Anspruch 5, wobei das Schmiermittel Mineralöl zur Grundlage hat.

7. Zusammensetzung nach Anspruch 4, wobei 0,001 bis 5,0 Gew.-% einer Verbindung der Formel I vorliegen, bezogen auf das Gewicht des organischen Materials.

8. Zusammensetzung nach Anspruch 7, wobei 0,02 bis 1,0 Gew.-% einer Verbindung der Formel I vorliegen, bezogen auf das Gewicht des organischen Materials.

9. Zusammensetzung nach Anspruch 4, umfassend eines oder mehrere zusätzliche Additive, die zur Verbesserung der Betriebseigenschaften des organischen Materials bekannt sind.

10. Zusammensetzung nach Anspruch 9, wobei das orgarische Material ein Schmiermittel ist und das zusätzliche Additiv eines oder mehrere eines weiteren Antioxidans, eines weiteren Metalldesaktivators, eines Rosthemmers, eines Viskositätsindexverbesserers, eines Stockpunkterniedrigers, eines Dispersants/Tensids und eines Antiverschleißadditivs ist.

11. Zusammensetzung nach Anspruch 10, wobei das weitere Additiv ein phenolisches oder ein Antioxidans vom Amintyp umfaßt.

12. Zusammensetzung nach Anspruch 11, wobei das zusätzliche Additiv Diphenylamin, octyliertes Diphenylamin, N-Phenyl-1-naphthylamin oder N-(octyliertes Phenyl)-1-naphthylamin ist.

**Patentansprüche für folgenden Vertragsstaat : ES**

1. Verfahren zur Herstellung einer Triazolverbindung der Formel I

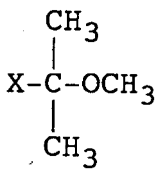

(I)

worin X den Triazolrest der Formel II

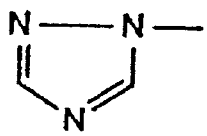

(II)

oder den Benzotriazolrest der Formel III oder IIIA bedeutet

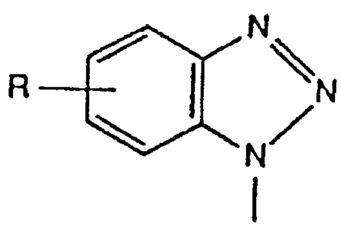 (III) 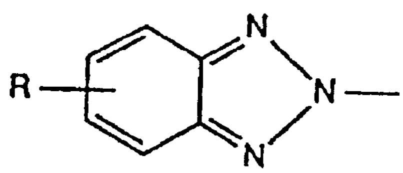 (IIIA)

worin R Wasserstoff oder Methyl bedeutet, umfassend die Umsetzung von 2-Methoxypropen der Formel IV:

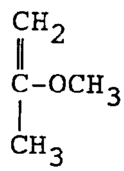

(IV)

mit einer Triazolverbindung der Formel V oder VI:

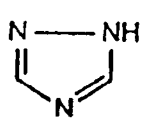 (V) 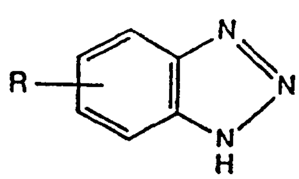 (VI)

worin R wie vorstehend definiert ist, in Gegenwart eines sauren Katalysators.

2. Verfahren nach Anspruch 1, wobei der saure Katalysator Schwefelsäure, Phosphorsäure, ein saures Ionenaustauscherharz, Bentonit, Montmorillonit, Fuller's Erde oder p-Toluolsulfonsäure ist.

3. Zusammensetzung, umfassend ein organisches Material und als Metalldesaktivator und/oder Antioxidans mindestens eine Verbindung der Formel I nach Anspruch 1.

4. Zusammensetzung nach Anspruch 3, wobei das organische Material ein Schmiermittel auf der Grundlage von Mineralöl oder synthetischen Ölen ist.

5. Zusammensetzung nach Anspruch 4, wobei das Schmiermittel Mineralöl zur Grundlage hat.

6. Zusammensetzung nach Anspruch 3, wobei 0,001 bis 5,0 Gew.-% einer Verbindung der Formel I vorliegen, bezogen auf das Gewicht des organischen Materials.

7. Zusammensetzung nach Anspruch 6, wobei 0,02 bis 1,0 Gew.-% einer Verbindung der Formel I vorliegen, bezogen auf das Gewicht des organischen Materials.

8. Zusammensetzung nach Anspruch 3, umfassend eines oder mehrere zusätzliche Additive, die zur Verbesserung der Betriebseigenschaften des organischen Materials bekannt sind.

9. Zusammensetzung nach Anspruch 8, wobei das organische Material ein Schmiermittel ist und das zusätzliche Additiv eines oder mehrere eines weiteren Antioxidans, eines weiteren Metalldesaktivators, eines Rosthemmers, eines Viskositätsindexverbesserers, eines Stockpunkterniedrigers, eines Dispersants/Tensids und eines Antiverschleißadditivs ist.

10. Zusammensetzung nach Anspruch 9, wobei das weitere Additiv ein phenolisches oder ein Antioxidans vom Amintyp umfaßt.

11. Zusammensetzung nach Anspruch 10, wobei das zusätzliche Additiv Diphenylamin, octyliertes Diphenylamin, N-Phenyl-1-naphthylamin oder N-(octyliertes Phenyl)-1-naphthylamin ist.

## Revendications

**Revendications pour les Etats contractants suivants : BE, DE, FR, GB, IT, NL**

1. Composé triazole ayant la formule I

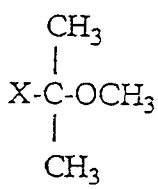

(I)

dans laquelle X est le résidu triazole de formule II

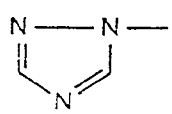

(II)

ou le résidu benzotriazole de formule III ou IIIA

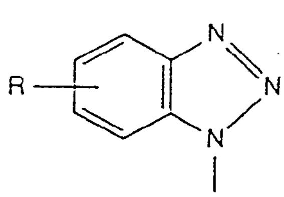

(III)

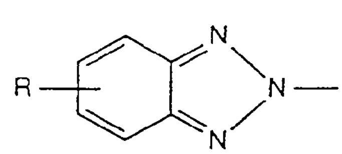

(IIIA)

dans laquelle R est un atome d'hydrogène ou un groupe méthyle.

2. Procédé pour la production d'un composé triazole de formule I, tel que défini dans la revendication 1, comprenant le réaction, en présence d'un catalyseur acide, du 2-méthoxypropène de formule IV :

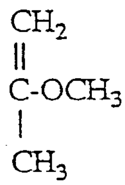 (IV)

avec un composé triazole ayant la formule V ou VI :

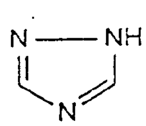 (V) 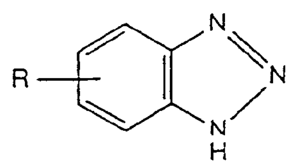 (VI)

dans laquelle R est tel que défini dans la revendication 1.

3. Procédé selon la revendication 2, dans lequel le catalyseur acide est l'acide sulfurique, l'acide phosphorique, une résine échangeuse d'ions acide, la bentonite, la montmorillonite, la terre de Foulon ou l'acide p-toluènesulfonique.

4. Composition comprenant une matière organique et en tant que désactivateur de métaux et/ou antioxydant, au moins un composé ayant la formule I, tel que défini dans la revendication 1.

5. Composition selon la revendication 4, dans laquelle la matière organique est un lubrifiant à base d'une huile minérale ou d'huiles synthétiques.

6. Composition selon la revendication 5, dans laquelle le lubrifiant est à base d'huile minérale.

7. Composition selon la revendication 4, dans laquelle 0,001 à 5,0% en poids d'un composé de formule I est présent, par rapport au poids de la matière organique.

8. Composition selon la revendication 7, dans laquelle 0,02 à 1,0% en poids d'un composé de formule I est présent, par rapport au poids de la matière organique.

9. Composition selon la revendication 4, comprenant un ou plusieurs additifs supplémentaires connus pour améliorer les propriétés de fonctionnement de la matière organique.

10. Composition selon la revendication 9, dans laquelle la matière organique est un lubrifiant et l'additif supplémentaire est un ou plusieurs d'un antioxydant supplémentaire, d'un désactivateur de métaux supplémentaire, d'un inhibiteur de rouille, d'un agent d'amélioration de l'indice de viscosité, d'un agent de diminution du point d'écoulement, d'un dispersant/tensio-actif et d'un additif anti-usure.

11. Composition selon la revendication 10, dans laquelle l'additif supplémentaire comprend un antioxydant phénolique ou de type amine.

12. Composition selon la revendication 11, dans laquelle l'additif supplémentaire est la diphénylamine, la diphénylamine octylée, la N-phényl-1-naphtylamine ou la N-(phényl octylé)-1-naphtylamine.

**Revendications pour l'Etat contractant suivant : ES**

1. Procédé pour la production d'un composé triazole de formule I,

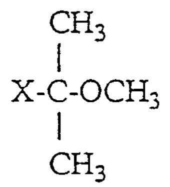

(I)

dans laquelle X est le résidu triazole de formule II

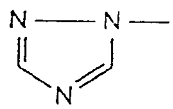

(II)

ou le résidu benzotriazole de formule III ou IIIA

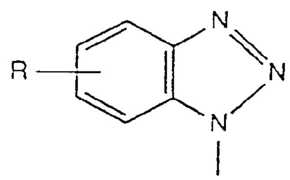 (III) 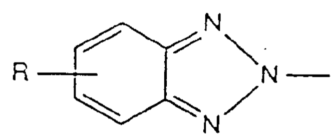 (IIIA)

dans laquelle R est un atome d'hydrogène ou un groupe méthyle, comprenant la réaction, en présence d'un catalyseur acide, du 2-méthoxypropène de formule IV :

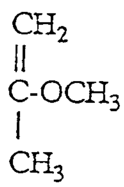

(IV)

avec un composé triazole ayant la formule V ou VI :

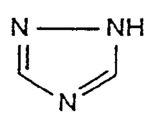 (V) 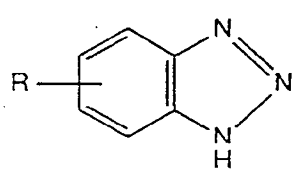 (VI)

dans laquelle R est tel que défini ci-dessus.

2. Procédé selon la revendication 1, dans laquelle le catalyseur acide est l'acide sulfurique, l'acide phosphorique, une résine échangeuse d'ions acide, la bentonite, la montmorillonite, la terre de Foulon ou l'acide p-toluènesulfonique.

3. Composition comprenant une matière organique et en tant que désactivateur des métaux et/ou antioxydant, au moins un composé ayant la formule I, tel que défini dans la revendication 1.

4. Composition selon la revendication 3, dans laquelle la matière organique est un lubrifiant à base d'huile minérale ou d'huiles synthétiques.

5. Composition selon la revendication 4, dans laquelle le lubrifiant est à base d'huile minérale.

**6.** Composition selon la revendication 3, dans laquelle 0,001 à 5% en poids d'un composé de formule I est présent, par rapport au poids de la matière organique.

**7.** Composition selon la revendication 6, dans laquelle 0,02 à 1,0% en poids d'un composé de formule I est présent, par rapport au poids de la matière organique.

**8.** Composition selon le revendication 3, comprenant un ou plusieurs additifs supplémentaires connus pour améliorer les propriétés de fonctionnement de la matière organique.

**9.** Composition selon la revendication 8, dans laquelle la matière organique est un lubrifiant et l'additif supplémentaire est un ou plusieurs d'un antioxydant supplémentaire, d'un désactivateur de métaux supplémentaire, d'un inhibiteur de rouille, d'un agent d'amélioration de l'indice de viscosité, d'un agent de diminution du point d'écoulement, d'un dispersant/tensio-actif et d'un additif anti-usure.

**10.** Composition selon la revendication 9, dans laquelle l'additif supplémentaire comprend un antioxydant phénolique ou de type amine.

**11.** Composition selon la revendication 10, dans laquelle l'additif supplémentaire est la diphénylamine, la diphénylamine octylée, la N-phényl-1-naphtylamine ou la N-(phényl octylé)-1-naphtylamine.